# EUROPÄISCHE PATENTSCHRIFT

(11) **EP 3 506 861 B1**
(45) Veröffentlichungstag und Bekanntmachung des Hinweises auf die Patenterteilung: **29.03.2023**
(21) Anmeldenummer: 17761266.0
(22) Anmeldetag: 04.09.2017
(51) Int. Cl.: A61F 9/00

(54) **INJEKTOR FÜR AUGENIMPLANTAT**
INJECTOR FOR OCULAR IMPLANT
INJECTEUR POUR UN IMPLANT OCULAIRE

(30) Priorität: 02.09.2016 DE 102016116507
(43) Veröffentlichungstag der Anmeldung: 10.07.2019
(73) Patentinhaber: Implandata Ophthalmic Products GmbH, 30159 Hannover (DE)
(72) Erfinder: OSTERMEIER, Max, 21218 Seevetal (DE); MEYER, Stefan, 30173 Hannover (DE)
(74) Vertreter: VKK Patentanwälte PartG mbB
(86) Internationale Anmeldenummer: PCT/EP2017/072094
(87) Internationale Veröffentlichungsnummer: WO 2018/042033

(56) Entgegenhaltungen:
- US-A1- 2001 020 171
- US-A1- 2008 119 865
- US-A1- 2010 331 868

## Beschreibung

Die Erfindung betrifft einen Injektor für die Implantation eines Sensorimplantats im menschlichen oder tierischen Auge, nämlich zur suprachoroidalen Implantation eines Drucksensors für die drahtlose Messung des Augeninnendrucks.

Es sind Injektoren für Linsenimplantate im Zusammenhang mit Kataraktoperationen bekannt, bei denen die zu implantierende künstliche Linse gefaltet und durch einen schmalen Kanal in eine Inzision des Auges eingebracht wird. Diese bekannten Injektoren sind jedoch für die suprachoroidale Implantation von Sensorimplantaten nicht geeignet. Üblicherweise werden solche Sensorimplantate mittels einer Pinzette in eine vorher in den Augapfel eingebrachte Inzision eingeschoben, welches mit Risiken für den Patienten verbunden ist, denn es besteht dabei die Gefahr einer Beschädigung der Choriodea mit unkontrollierbaren Blutungen.

Die veröffentlichte US-Patentanmeldung US 2007/0182725 A1 hat ein Verfahren zur Herstellung eines Instruments zur subkutanen Implantation von medizinischen Geräten, wie beispielsweise Sensoren zur Erfassung von medizinisch relevanten Daten im Körper. Das Instrument setzt sich aus einem eingekerbten Körper (incised body), also einem Hohlkörper, der eine nicht-kreisrunde koaxiale Bohrung aufweist und einem zweiteiligen Stößel (Stempel), zusammen. Der Stößel weist ein flaches und dünnes Zungenblatt auf. Durch Drücken des Stößels kann ein Sensor durch die Bohrung des Hohlkörpers als Implantat in den Körper injiziert werden.

In US 2008/0119865 A1 ist eine Vorrichtung zum Laden einer intraokularen Linse in eine Injektionspatrone offenbart. Außerdem ist eine Injektionspatrone zur Aufnahme einer flexiblen intraokularen Linse offenbart. Beide offenbarten Vorrichtungen beschäftigen sich somit mit der Vorbereitung einer intraokularen Linse für eine spätere Injektion in ein Auge eines Lebewesens. Die Injektion in den Körper eines Lebewesens selbst wird in US 2008/0119865 A1 nicht beschrieben.

Die US-Patentanmeldung US 2001/0020171 A1 offenbart eine Vorrichtung zum Injizieren einer flexiblen intraokularen Linse in ein Auge. Die Vorrichtung umfasst ein röhrenförmiges Teil und einen beweglichen Stößel, der in den Kanal aufgenommen ist. Der Kanal hat ein offenes distales Ende, das angepasst ist, für die Implantation der Linse in einem Auge aufgenommen zu werden. Das röhrenförmige Teil weist ferner eine Öffnung auf, um die Linse in den Bereitstellungsbereich einzuführen. Die Öffnung zum Einführen der Linse in den Bereitstellungsbereich ist mit einer beweglichen Abdeckung verschließbar. Am distalen Ende des Stößels ist ein Sichtindikator, um den Chirurgen anzuzeigen, ob die distale Spitze des Stößels ungenau im Bereitstellungsbereich angeordnet ist bevor die Linse geladen wird.

Des Weiteren muss das Sensorimplantat in einer bestimmten Orientierung in eine künstlich geschaffene Tasche zwischen Sklera und Choroidea eingebracht werden, damit der auf einer Seite des Sensorimplantats angebrachte Drucksensor den korrekten Augeninnendruck messen kann.

Aufgabe der Erfindung ist es daher, einen Injektor für die Implantation eines Sensorimplantats im menschlichen oder tierischen Auge anzugeben, mit dem ein übliches Sensorimplantat schnell, traumaarm und materialschonend in eine suprachoroidale Tasche des Auges implantierbar ist.

Die Aufgabe wird gelöst durch einen Injektor mit den Merkmalen des Anspruchs 1.

Ein Injektor zur Aufnahme des Sensorimplantats besitzt eine rohrförmige Injektionskammer, deren innere Wandflächen einen nicht rotationssymmetrischen Querschnitt ovalen oder rechteckigen Querschnitt aufweisen, wobei der Injektor einen Schieber aufweist, mit dem das Sensorimplantat aus der Injektionskammer hinaus durch die Injektionsöffnung gedrückt werden kann. Die Injektionskammer ist an einem freien Ende mit einer Injektionsöffnung versehen, durch die das Sensorimplantat während der Implantation herausgleiten und in eine Inzision des Auges hineingleiten kann, wobei die inneren Wandflächen der Injektionskammer den ebenfalls nicht rotationssymmetrischen Querschnitt des Sensorimplantats umschließen und eine Drehung des Sensorimplantats um eine in Injektionsrichtung liegende Drehachse verhindern und wobei ein zwischen Injektionsöffnung und dem Sensorimplantat liegender erster Kammerabschnitt der Injektionskammer mit einem Viskoelastikum gefüllt ist und wobei das zunächst in einem von der Injektionsöffnung weiter entfernten zweiten Kammerabschnitt der Injektionskammer platzierte Sensorimplantat durch den gefüllten ersten Kammerabschnitt hindurch aus der Injektionskammer hinausdrückbar ist.

Übliche Gehäuse solcher Sensorimplantate haben eine flache längliche Form wie beispielsweise ein Ellipsoid oder ein Quader mit abgerundeten Ecken und Kanten. Befindet sich ein solches Sensorimplantat in der rohrförmigen Injektionskammer, so ist seine Längsachse im Wesentlichen parallel zur Längsachse der Injektionskammer und damit zur Injektionsrichtung ausgerichtet. Das Sensorimplantat kann sich daher in der Injektionskammer nicht um eine beliebige Achse drehen, die senkrecht auf der Injektionsrichtung steht. Wegen seines nicht rotationssymmetrischen Querschnitts und des ebenfalls nicht rotationssymmetrischen Querschnitts der inneren Wandflächen der Injektionskammer kann sich das Sensorimplantat aber auch nicht um eine in Injektionsrichtung liegende Achse drehen, denn es ist im Querschnitt "flach", das heißt seine Breite ist größer als seine Dicke.

Durch diese flache Form wird eine suprachoroidale Implantation erst möglich, denn eine zwischen Sklera und Choroidea auszubildende Tasche kann nur eine begrenzte Breite haben, weil ansonsten beim Patienten Beschwerden auftreten würden. Eine typische Dicke des Sensorimplantats liegt bei 2 mm oder darunter. Seine Breite ist vorzugsweise kleiner als 3,5 mm und die Länge liegt typischerweise bei 7 mm oder weniger. Der Drucksensor ist üblicherweise in einer der beiden großen Flächen des Sensorimplantats untergebracht. Die mit dem Drucksensor versehene Fläche muss beim implantierten Sensorimplantat dem Augenzentrum und damit der Choroidea zugewandt sein, damit der Drucksensor den Augeninnendruck korrekt messen kann. Wäre die mit dem Drucksensor versehene Seite des Sensorimplantats der Augenaußenseite und damit der Sklera zugewandt, so wären die gewonnenen Messwerte nicht mit dem Augeninnendruck korreliert. Die korrekte Lage des Sensorimplantats in der subchoroidalen Tasche des Auges ist also entscheidend für korrekte Messwerte.

Der erfindungsgemäße Injektor hat den Vorteil, dass das Sensorimplantat stets in korrekter Lage implantiert wird und dass die Implantation im Vergleich mit der Verwendung einer Pinzette schneller und traumaarm erfolgt.

Der Injektor weist einen Schieber auf, mit dem das Sensorimplantat aus der Injektionskammer hinaus durch die Injektionsöffnung gedrückt werden kann. Durch die Verbindung von Injektor und Schieber steht dieser stets in der korrekten Position zur Verfügung.

In vorteilhafter Ausgestaltung der Erfindung sind die Hohlräume zwischen den inneren Wandflächen der Injektionskammer und dem Sensorimplantat mit einem Viskoelastikum, vorzugsweise mit Hyaluronsäure gefüllt. Das Viskoelastikum dient dabei als Schmiermittel zur Verringerung der Reibung zwischen den inneren Wandflächen der Injektionskammer und dem Sensorimplantat. Außerdem bleiben Teile des Viskoelastikums am Sensorimplantat haften, wenn dies die Injektionsöffnung verlässt und in die Inzision des Auges hineingleitet, wobei auch im Auge die Reibung vermindert und das Gleiten erleichtert wird.

Erfindungsgemäß ist ein zwischen der Injektionsöffnung und dem Sensorimplantat liegender erster Kammerabschnitt der Injektionskammer mit dem Viskoelastikum, vorzugsweise mit Hyaluronsäure gefüllt und das zunächst in einem von der Injektionsöffnung weiter entfernten, zweiten Kammerabschnitt der Injektionskammer platzierte Sensorimplantat kann durch den gefüllten ersten Kammerabschnitt hindurch aus der Injektionskammer hinaus gedrückt werden. Dabei taucht das Sensorimplantat in das Viskoelastikum ein und wird komplett davon benetzt, bevor es die Injektionsöffnung verlässt.

In einer vorteilhaften Weiterbildung der Erfindung ist vorgesehen, dass der zweite Kammerabschnitt als Ladekammer mit einer seitlichen Ladeöffnung zum Einlegen des Sensorimplantats ausgestaltet ist. Die "trockene" Ladekammer, kann mittels der Ladeöffnung geöffnet werden, ohne dass die Gefahr des Austritts von Viskoelastikum besteht. Nach dem Einlegen des Sensorimplantats in die Ladekammer und dem Schließen der Ladeöffnung wird das Sensorimplantat dann mittels des Schiebers in den mit Viskoelastikum gefüllten zweiten Kammerabschnitt geschoben und dabei durch das Viskoelastikum benetzt, bevor es durch die Injektionsöffnung hinaus gedrückt wird.

Die Erfindung kann noch verbessert werden durch die Maßnahme, dass der Schieber mit einer umlaufenden Dichtlippe versehen ist, um das Sensorimplantat mithilfe des Viskoelastikums hydraulisch aus der Injektionskammer hinauszudrücken. Im Idealfall braucht daher der Schieber das Sensorimplantat nicht einmal zu berühren, denn er drückt das Viskoelastikum, welches hier als Hydraulikflüssigkeit dient, in Richtung der Injektionsöffnung und aus dieser heraus, wobei das Viskoelastikum das Sensorimplantat mitnimmt. Bei dieser Ausführungsform ist es auch möglich, zunächst ein gewisses Volumen des Viskoelastikums in die Sklerainzision des Auges einzupressen, um auf diese Weise erst eine subchoroidale Tasche zwischen Sklera und Choroidea zu schaffen, in der dann das Sensorimplantat aufgenommen wird.

Um ein Aufsteigen oder Verrutschen des in die Ladekammer eingelegten Implantats bei Betätigung des Schiebers zu vermeiden, wird empfohlen, dass der Schieber im Bereich der Dichtlippe mit einem in Injektionsrichtung vorspringenden Stopper versehen ist.

Um das Auftreten eines zu starken Überdrucks in der suprachoroidalen Kavität während der Implantation zu vermeiden, wird eine Ausführungsform der Erfindung empfohlen, bei der die Injektionskammer mit seitlichen Ausströmöffnungen für das Viskoelastikum versehen ist. Bei gegebener Vorschubgeschwindigkeit des Schiebers hängt der maximal erzeugte Druck in der suprachoroidalen Kavität im Wesentlichen vom Querschnitt und der Anzahl der Ausströmöffnungen ab und kann somit durch Wahl der Parameter nach oben hin begrenzt werden.

Ein weiteres bevorzugtes Ausgestaltungsmerkmal der Erfindung besteht darin, dass im Bereich der Injektionsöffnung ein in Injektionsrichtung vorspringender Trokar mit zu seinem freien Ende hin schräg aufeinander zulaufenden Trokarrändern angeordnet ist. Während der Implantation wird der Trokar als Führungsspitze in die vorbereitete Sklerainzision eingeführt.

Damit wird die Injektionsöffnung des Injektors im Verhältnis zur Sklerainzision korrekt positioniert.

Der Trokar kann mit abgerundeten Trokarrändern atraumatisch ausgeführt sein. In diesem Fall muss die Sklera vor der Implantation in der erforderlichen Breite eröffnet werden.

Der Trokar kann aber auch mit scharfkantigen Trokarrändern versehen sein, mittels derer eine zunächst kleine Sklerainzision auf die benötigte Breite erweitert werden kann. In diesem Fall wäre aber die Trokarspitze zur Vermeidung ungewollter Verletzungen des Auges abgerundet ausgeführt.

Die Trokarspitze könnte auch zur Sklerainnenfläche hin gebogen ausgeführt sein, um eine versehentliche Punktion der Choroidea zu vermeiden.

Alle genannten Ausführungsformen des Trokars können vorteilhaft mit dem erfindungsgemäßen Injektor verwirklicht sein.

Vorteilhaft ist auch eine Ausführungsform der Erfindung, bei der der Injektor mit vorgeladenem Sensorimplantat ausgestaltet ist. Bei dieser Ausführungsform kann es keine Fehler hinsichtlich der Lage des Sensorimplantats beim Einfügen in den Injektor geben.

Nach einer besonders vorteilhaften Ausführungsform ist der Injektor sterilisiert.

Diese Ausführungsform hat den Vorteil, dass das versehentliche Einbringen von Keimen in den Injektor ausgeschlossen ist.

Ausführungsbeispiele der Erfindung werden nachfolgend anhand der Zeichnungen näher erläutert. Die Figuren zeigen im Einzelnen:
- Figur 1:: einen erfindungsgemäßen Injektor in einer perspektivischen Darstellung mit einem Sensorimplantat vor dem Einlegen in den Injektor;
- Figur 2:: wie Figur 1, jedoch mit in den Injektor eingelegtem Sensorimplantat;
- Figur 3:: ein vergrößertes Detail des Injektors von Figur 1;
- Figur 4:: einen Schieber des Injektors von Figur 1 ohne Dichtlippe;
- Figur 5:: ein vergrößertes Detail eines Schiebers mit Dichtlippe;
- Figur 6:: ein vergrößertes Detail eines Schiebers mit Dichtlippe und Stopper.

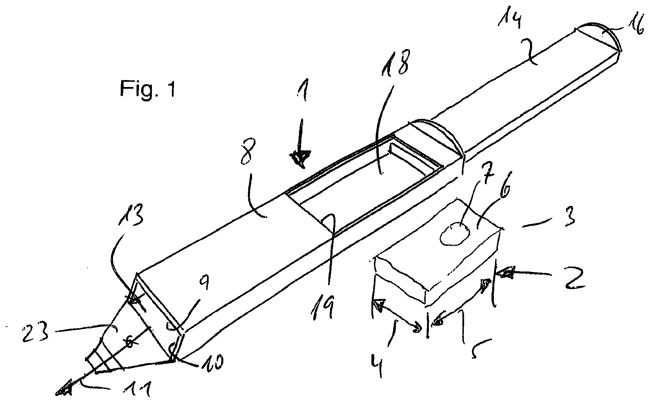

In Figur 1 erkennt man einen Injektor 1 zur Implantation eines Sensorimplantat 2 in eine suprachoroidale Tasche des Auges. Das Sensorimplantat 2 dient zur Messung des Augeninnendrucks. Es hat die Form eines Quaders mit einer relativ geringen Dicke 3, die vorzugsweise weniger als 2 mm beträgt, einer Breite 4, die etwa 3,5 mm oder weniger beträgt, und einer Länge 5, die kleiner als 7 mm sein sollte. An einer seiner großen Seitenflächen 6 besitzt das Sensorimplantat 2 einen Drucksensor 7, der nach der Implantation in eine suprachoroidale Tasche des Auges an der Choroidea anliegen muss, um den Augeninnendruck korrekt zu messen.

Der Injektor 1 besitzt eine rohrförmige Injektionskammer 8, deren innere Wandflächen 9, 10 einen nicht rotationssymmetrischen Querschnitt aufweisen, nämlich einen rechteckigen Querschnitt mit zwei gegenüberliegenden breiten Wandflächen 9 und zwei gegenüberliegenden schmalen Wandflächen 10. Die Injektionskammer 8 dient zur Aufnahme des Sensorimplantats 2, welches ebenfalls einen rechteckigen Querschnitt mit einer größeren Breite 4 und einer kleineren Dicke 3 aufweist. Die Breite 4 des Sensorimplantats ist etwas geringer als die Abmessung der breiten Wandfläche 9 und die Dicke 3 des Sensorimplantats ist etwas geringer als die Abmessung der schmalen Wandfläche 10, so dass die inneren Wandflächen 9, 10 der Injektionskammer 8 das Sensorimplantat 2 mit Spiel umschließen. Dadurch ist das Sensorimplantat 2 innerhalb der rohrförmigen Injektionskammer 8 in Injektionsrichtung 11 leicht verschiebbar. Andererseits verhindern die nicht rotationssymmetrischen Querschnitte der Injektionskammer 8 einerseits und des Sensorimplantats 2 andererseits eine Drehung des Sensorimplantats 2 um eine in Injektionsrichtung 11 liegende Achse. Da das Sensorimplantat 2 eine Länge 5 besitzt, die größer ist als die breiten Wandflächen 9 der Injektionskammer 8, kann sich das Sensorimplantat innerhalb der Injektionskammer 8 auch nicht um eine senkrecht zur Injektionsrichtung 11 weisende Drehachse 12 oder um eine beliebige andere Drehachse drehen.

Die Injektionskammer 8 besitzt an ihrem freien Ende eine Injektionsöffnung 13, durch die hindurch das Sensorimplantat 2 während der Implantation in Injektionsrichtung 11 hindurchtreten und aus der Injektionskammer 8 hinaus in die nicht gezeigte Sklerainzision des Auges gedrückt werden kann. Zum Herausdrücken des Sensorimplantats 2 aus der Injektionskammer 8 dient ein Schieber 14, der nach Art einer Injektionsspritze am Injektor 1 gleitend gelagert ist. Während der Implantation wird der Schieber 14 in die Injektionskammer 8 hineingedrückt, wobei er das Sensorimplantat 2 aus der Injektionsöffnung 3 herausschiebt. Der Schieber 14 kann manuell, durch eine geeignete Mechanik oder motorisch betätigt werden.

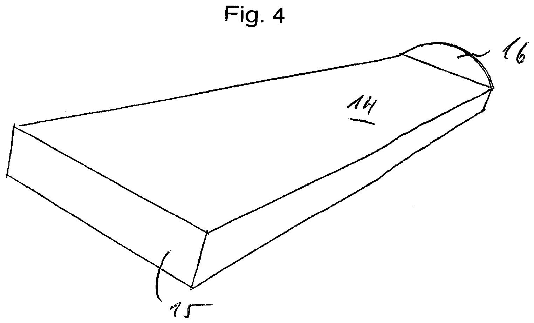

In der vergrößerten Darstellung von Figur 4 erkennt man die quaderförmige Gestalt des Schiebers mit einer schmalen Stirnfläche 15, deren rechteckiger Querschnitt an die Abmessungen der inneren Wandflächen 9, 10 der Injektionskammer 8 angepasst sind. In einer ersten Ausführungsform dient die schmale Stirnfläche 15 auch als Kontaktfläche zum Sensorimplantat 2, um es durch die Injektionskammer 8 hindurch aus der Injektionsöffnung 13 hinauszudrücken. Das andere Ende des quaderförmigen Schiebers 14 ist mit einer Verbreiterung 16 versehen, welche die manuelle Betätigung des Schiebers 14 erleichtert.

Zur Verringerung der Reibung zwischen den inneren Wandflächen 9, 10 des Injektors, einerseits und den Außenflächen des Sensorimplantats 2 andererseits ist die Injektionskammer 8 mit einem Viskoelastikum, im vorliegenden Fall mit Hyaluronsäure versehen, welche die Hohlräume zwischen den inneren Wandflächen 9, 10 und den Außenflächen des Sensorimplantats 2 ausfüllt und als Schmiermittel dient.

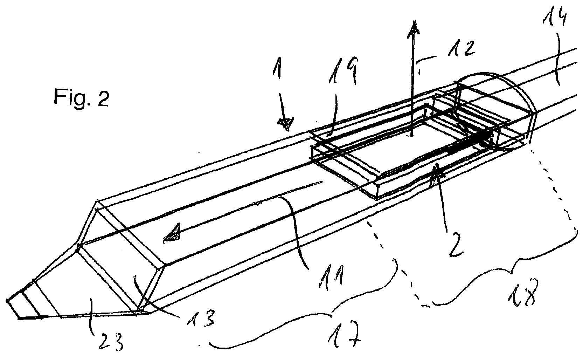

In einer zweiten Ausführungsform der Erfindung ist ein zwischen der Injektionsöffnung 13 und dem Sensorimplantat 2 liegender erster Kammerabschnitt 17 der Injektionskammer 8 mit Hyaluronsäure gefüllt. Ein benachbarter zweiter Kammerabschnitt ist als Ladekammer 18 ausgestaltet. Siehe hierzu insbesondere Figur 2. Die Ladekammer 18 besitzt eine Ladeöffnung 19, durch die hindurch das Sensorimplantat 2 in die Ladekammer 18 eingelegt werden kann. Danach kann das Sensorimplantat 2 durch den Schieber 14 aus der Ladekammer 18 in den ersten Kammerabschnitt 17 verschoben werden, wo es in die Hyaluronsäure eintaucht und davon benetzt wird, bevor es durch die Injektionsöffnung 13 austritt.

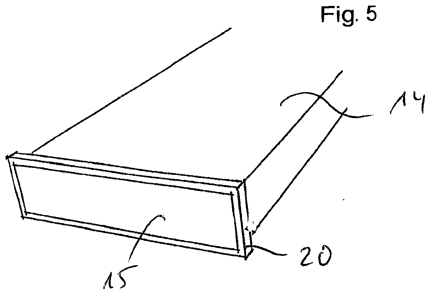

In einer in Figur 5 gezeigten zweiten Ausführungsform ist der Schieber 14 mit einer umlaufenden Dichtlippe 20 versehen, die den Schieber 14 beim Eintritt der Dichtlippe 20 in den ersten Kammerabschnitt 17 gegenüber den inneren Wandflächen 9, 10 der Injektionskammer 8 abdichtet, so dass das Sensorimplantat 2 mithilfe der Hyaluronsäure hydraulisch aus der Injektionskammer 8 hinausgedrückt werden kann.

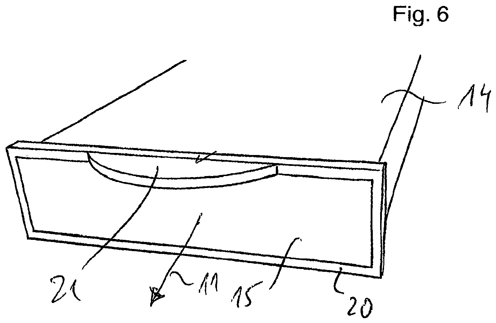

In einer in Figur 6 gezeigten weiteren Ausführungsform ist der Schieber 14 im Bereich der Dichtlippe 20 zusätzlich mit einem in Injektionsrichtung 11 vorspringenden Stopper 21 versehen, der ein Aufsteigen oder Verrutschen des in die Ladekammer 18 eingelegten Sensorimplantats bei Betätigung des Schiebers 14 verhindert.

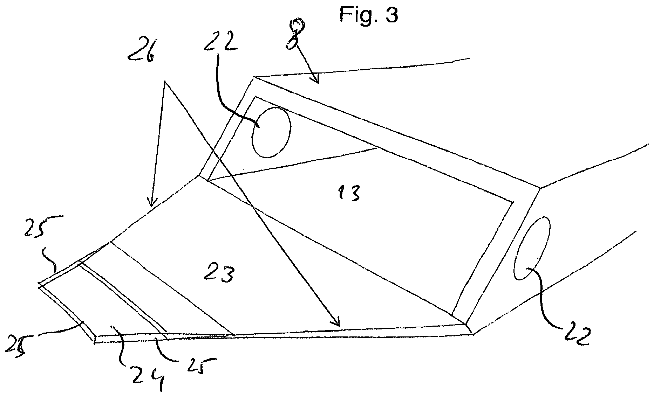

Wie man in Figur 3 erkennt, ist die Injektionskammer 8 mit zwei seitlichen Ausströmöffnungen 22 versehen, aus denen während der Betätigung des Schiebers 14 im Verlauf der Implantation überschüssige Hyaluronsäure austreten kann, damit der Druck der Hyaluronsäure in der suprachoroidalen Tasche des Auges nicht übermäßig groß wird.

Im Bereich der Injektionskammer 13 ist der Injektor 1 mit einem Trokar 23 versehen. Wie man am besten in Figur 3 erkennt, besitzt der Trokar 23 eine Trokarspitze 24, deren seitliche Kanten 25 abgerundet ausgestaltet sind, um während der Implantation ungewollte Verletzungen des Auges zu vermeiden. Die Trokarspitze 24 kann auch nach oben, also zur Sklerainnenfäche hin abgebogen sein, um eine versehentliche Punktion der Choroidea zu vermeiden. Des Weiteren besitzt der Trokar 23 schräg aufeinander zu laufende Trokarränder 26, die zur Vermeidung ungewollter Schnitte während der Implantation atraumatisch abgerundet ausgestaltet sein können. In einer abgewandelten Ausführungsform können die Trokarränder 26 aber auch scharfkantig ausgestaltet sein, um gegebenenfalls während der Implantation eine kleinere Sklerainzision so zu erweitern, dass das Sensorimplantat 2 hindurchpasst.

### BEZUGSZEICHENLISTE

- 1: Injektor
- 2: Sensorimplantat
- 3: Dicke
- 4: Breite
- 5: Länge
- 6: Seitenfläche
- 7: Drucksensor
- 8: Injektionskammer
- 9: breite Wandfläche
- 10: schmale Wandfläche
- 11: Injektionsrichtung
- 12: Drehachse
- 13: Injektionsöffnung
- 14: Schieber
- 15: schmale Stirnfläche
- 16: Verbreiterung
- 17: erster Kammerabschnitt
- 18: zweiter Kammerabschnitt / Ladekammer
- 19: Ladeöffnung
- 20: Dichtlippe
- 21: Stopper
- 22: Austrittsöffnungen
- 23: Trokar
- 24: Trokarspitze
- 25: Kanten
- 26: Trokarränder

## Patentansprüche

1. Injektor für die Implantation eines Sensorimplantats (2) im menschlichen oder tierischen Auge, nämlich zur suprachoroidalen Implantation eines Drucksensors für die drahtlose Messung des Augeninnendrucks, wobei der Injektor eine Injektionskammer (8) und einen Schieber (14) aufweist, mit dem ein Sensorimplantat (2) aus der Injektionskammer (8) hinaus durch eine Injektionsöffnung (13) gedrückt werden kann, **dadurch gekennzeichnet, dass** der Injektor (1) zur Aufnahme eines Sensorimplantats (2) eine rohrförmige Injektionskammer (8) besitzt, deren innere Wandflächen (9, 10) einen nicht rotationssymmetrischen ovalen oder rechteckigen Querschnitt aufweisen, dass die Injektionskammer (8) an einem freien Ende mit einer Injektionsöffnung (13) versehen ist, durch die ein Sensorimplantat (2) während der Implantation herausgleiten und in eine Sklerainzision des Auges hineingleiten kann, wobei die inneren Wandflächen (9, 10) der Injektionskammer (8) den ebenfalls nicht rotationssymmetrischen Querschnitt eines Sensorimplantats (2) umschließen und eine Drehung des Sensorimplantats (2) um eine in Injektionsrichtung (11) liegende Drehachse verhindern, und dass ein zwischen der Injektionsöffnung (13) und einem von der Injektionsöffnung (13) weiter entfernten zweiten Kammerabschnitt (18) der Injektionskammer (8) liegender erster Kammerabschnitt (17) der Injektionskammer (8) mit einem Viskoelastikum gefüllt ist und dass ein zunächst in dem zweiten Kammerabschnitt (18) platziertes Sensorimplantat (2) durch den gefüllten ersten Kammerabschnitt (17) hindurch aus der Injektionskammer (8) hinausdrückbar ist.

2. Injektor nach Anspruch 1, **dadurch gekennzeichnet, dass** das Viskoelastikum Hyaluronsäure ist.

3. Injektor nach einem der Ansprüche 1 oder 2, **dadurch gekennzeichnet, dass** der zweite Kammerabschnitt als Ladekammer (18) mit einer seitlichen Ladeöffnung (19) zum Einlegen des Sensorimplantats (2) ausgestaltet ist.

4. Injektor nach einem der Ansprüche 1 bis 3, **dadurch gekennzeichnet, dass** der Schieber (14) mit einer umlaufenden Dichtlippe (20) versehen ist, um das Sensorimplantat (2) mithilfe des Viskoelastikums hydraulisch aus der Injektionskammer 8 hinauszudrücken.

5. Injektor nach Anspruch 4, **dadurch gekennzeichnet, dass** der Schieber (14) im Bereich der Dichtlippe (20) mit einem in
Injektionsrichtung (11) vorspringenden Stopper (21) versehen ist.

6. Injektor nach Anspruch 4 oder 5, **dadurch gekennzeichnet, dass** die Injektionskammer (8) mit seitlichen Ausströmöffnungen (22) für das Viskoelastikum versehen ist.

7. Injektor nach einem der vorhergehenden Ansprüche, **dadurch gekennzeichnet, dass** im Bereich der Injektionsöffnung (13) ein in
Injektionsrichtung (11) vorspringender Trokar (23) mit zu seinem freien Ende hin schräg aufeinander zulaufenden Trokarrändern (26) und einer Trokarspitze (24) mit abgerundeten Kanten angeordnet ist.

8. Injektor nach einem der vorhergehenden Ansprüche, **dadurch gekennzeichnet, dass** der Injektor mit einem vorgeladenem Sensorimplantat (2) ausgestaltet ist.

9. Injektor nach Anspruch 8, **dadurch gekennzeichnet, dass** der Injektor sterilisiert ist.

## Claims

1. An injector for implanting a sensor implant (2) in a human or animal eye, namely for suprachoroidally implanting a pressure sensor for wirelessly measuring the internal eye pressure, the injector comprising an injection chamber (8) and a pusher (14), by means of which a sensor implant (2) can be pressed out of the injection chamber (8) through an injection opening (13), **characterized in that** the injector (1) comprises a tube-shaped injection chamber (8) for receiving a sensor implant (2), the inner wall surfaces (9, 10) thereof having a rotationally asymmetric oval or rectangular cross section, **in that** the injection chamber (8) has an injection opening (13) at a free end, through which a sensor implant (2) can slide out during implanting and can slide into a scleral incision in the eye, wherein the inner wall surfaces (9, 10) of the injection chamber (8) enclose the also rotationally asymmetric cross section of a sensor implant (2) and prevent rotating of the sensor implant (2) about an axis of rotation in the direction of injection (11), and **in that** a first chamber segment (17) of the injection chamber (8) present between the injection opening (13) and a second chamber segment (18) of the injection chamber (8) further away from the injection opening (13) is filled with a viscoelastic material, and **in that** a sensor implant (2) initially placed in the second chamber segment (18) can be pressed out of the injection chamber (8) through the filled, first chamber segment (17).

2. The injector according to claim 1, **characterized in that** the viscoelastic material is hyaluronic acid.

3. The injector according to any one of the claims 1 or 2, **characterized in that** the second chamber segment is implemented as a loading chamber (18) having a lateral loading opening (19) for inserting the sensor implant (2).

4. The injector according to any one of the claims 1 through 3, **characterized in that** the pusher (14) has a circumferential sealing lip (20) for hydraulically pressing the sensor implant (2) out of the injection chamber (8) by means of the viscoelastic material.

5. The injector according to claim 4, **characterized in that** the pusher (14) has a stopper (21) protruding in the injection direction (11) in the region of the sealing lip (20).

6. The injector according to claim 4 or 5, **characterized in that** the injection chamber (8) has lateral outlet openings (22) for the viscoelastic material.

7. The injector according to any one of the preceding claims, **characterized in that** a trocar (23) protruding in the injection direction (11) and having trocar edges (26) converging diagonally with each other toward the free end thereof and a having a trocar tip (24) having rounded edges is disposed in the region of the injection opening (13).

8. The injector according to any one of the preceding claims, **characterized in that** the injector comprises a preloaded sensor implant (2).

9. The injector according to claim 8, **characterized in that** the injector is sterilized.

## Revendications

1. Injecteur pour l'implantation d'un implant capteur (2) dans un oeil chez l'homme ou l'animal, à savoir pour l'implantation supra-choroïdienne d'un capteur de pression pour la mesure sans fil de la pression intra-oculaire, dans lequel l'injecteur présente une chambre d'injection (8) et un coulisseau (14), avec lequel un implant capteur (2) peut être poussé à travers une ouverture d'injection (13) hors de la chambre d'injection (8), **caractérisé en ce que** l'injecteur (1) possède, pour loger un implant capteur (2), une chambre d'injection tubulaire (8), dont les surfaces de paroi internes (9, 10) présentent une section transversale ovale ou rectangulaire non symétrique en rotation, que la chambre d'injection (8) est pourvue, sur une extrémité libre, d'une ouverture d'injection (13) par laquelle un implant capteur (2) peut sortir par glissement au cours de l'implantation et peut entrer par glissement dans une incision de la sclère de l'oeil, dans lequel les surfaces de paroi internes (9, 10) de la chambre d'injection (8) renferment la section transversale de la même manière non symétrique en rotation de l'implant capteur (2) et empêchent une rotation de l'implant capteur (2) autour d'un axe de rotation situé dans la direction d'injection (11), et qu'une première section de chambre (17) de la chambre d'injection (8), qui se situe entre l'ouverture d'injection (13) et une deuxième section de chambre (18) de la chambre d'injection (8) davantage éloignée de l'ouverture d'injection (13), est remplie d'un produit viscoélastique, et qu'un implant capteur (2) placé en premier lieu dans la deuxième section de chambre (18) peut être poussé hors de la chambre d'injection (8) en passant la première section de chambre (17) remplie.

2. Injecteur selon la revendication 1, **caractérisé en ce que** le produit viscoélastique est de l'acide hyaluronique.

3. Injecteur selon l'une quelconque des revendications 1 ou 2, **caractérisé en ce que** la deuxième section de chambre est configurée en tant que chambre de chargement (18) avec une ouverture de chargement latérale (19) pour y placer l'implant capteur (2).

4. Injecteur selon l'une quelconque des revendications 1 à 3, **caractérisé en ce que** le coulisseau (14) est pourvu d'une lèvre d'étanchéité (20) périphérique pour pousser de manière hydraulique l'implant capteur (2) hors de la chambre d'injection (8) à l'aide du produit viscoélastique.

5. Injecteur selon la revendication 4, **caractérisé en ce que** le coulisseau (14) est pourvu dans la zone de la lèvre d'étanchéité (20) d'un bouchon (21) faisant saillie dans la direction d'injection (11).

6. Injecteur selon la revendication 4 ou 5, **caractérisé en ce que** la chambre d'injection (8) est pourvue d'ouvertures d'évacuation latérales (22) pour le produit viscoélastique.

7. Injecteur selon l'une quelconque des revendications précédentes, **caractérisé en ce qu'**un trocart (23) faisant saillie dans la direction d'injection (11) est disposé par des bords de trocart (26) convergeant les uns vers les autres à l'oblique en direction de son extrémité libre et par une pointe de trocart (24) avec des bords arrondis dans la zone de l'ouverture d'injection (13).

8. Injecteur selon l'une quelconque des revendications précédentes, **caractérisé en ce que** l'injecteur est configuré avec un implant capteur (2) préchargé.

9. Injecteur selon la revendication 8, **caractérisé en ce que** l'injecteur est stérilisé.
